# EUROPEAN PATENT SPECIFICATION

(11) **EP 3 604 550 B1**
(45) Date of publication and mention of the grant of the patent: **29.11.2023**
(21) Application number: 17902332.0
(22) Date of filing: 29.05.2017
(51) Int. Cl.: C12Q 1/48, C12Q 1/54, G01N 33/66

(54) **METHOD FOR DETECTING ATP BY USING PERSONAL BLOOD GLUCOSE METER**
VERFAHREN ZUM NACHWEIS VON ATP DURCH VERWENDUNG EINES PERSÖNLICHEN BLUTZUCKERMESSERS
PROCÉDÉ DE DÉTECTION D'ATP À L'AIDE UN GLYCOMÈTRE INDIVIDUEL

(30) Priority: 23.03.2017 KR 20170036737
(43) Date of publication of application: 05.02.2020
(73) Proprietor: Korea Advanced Institute of Science and Technology, Daejeon 34141 (KR)
(72) Inventor: PARK, Hyun Gyu, Daejeon 34141 (KR); AHN, Jun Ki, Daejeon 34141 (KR); KIM, Hyo Yong, Daejeon 34141 (KR); JU, Yong, Daejeon 34141 (KR)
(74) Representative: Mewburn Ellis LLP
(86) International application number: PCT/KR2017/005563
(87) International publication number: WO 2018/174336

(56) References cited:
- WO-A1-2013/038183
- WO-A2-2015/069872
- WO-A2-2015/069872
- JP-A- S6 423 900
- JP-A- 2001 204 496
- US-A1- 2016 252 515
- US-B2- 7 122 333
- US-B2- 8 945 943
- YU XIANG ET AL: "Using personal glucose meters and functional DNA sensors to quantify a variety of analytical targets", NATURE CHEMISTRY, vol. 3, no. 9, 24 July 2011 (2011-07-24), pages 697-703, XP055235002, London ISSN: 1755-4330, DOI: 10.1038/nchem.1092

## Description

### [Technical Field]

The present invention relates to a method of detecting or quantifying ATP in a sample, the method comprising:
(a) adding a sample to be detected to a composition for detecting ATP comprising glucose, NADP, phosphoenolpyruvic acid, hexokinase, and pyruvate kinase, and then converting glucose in the sample into glucose-6-phosphate; and
(b) measuring a concentration of the glucose using a blood glucose meter to detect or quantify ATP in the sample.

### [Background Art]

As awareness about food cleanliness and safety has improved, hygienic conditions are increasingly monitored in food providers or catering companies serving schools and public institutions to detect food poisoning and pathogenic microorganisms present in various foods. Currently, the most common method of detecting a microorganism is technology for detecting ATP, which is a metabolite of microorganisms, using an optical analysis method.

ATP is used as a coenzyme in various intracellular reactions catalyzed by enzymes to produce sugars, and plays an important role in many aspects, such as energy transfer to cells and the like. Thus, ATP detection may enable cleanliness monitoring by confirming whether dishes, restaurant facilities, and the like are contaminated by microorganisms, and may also be used in drug screening and toxicological stability tests.

Among currently developed ATP detection techniques, a method using luciferase extracted from fireflies is most widely used. Specifically, this method is a technique for analyzing ATP in a sample by measuring light having a wavelength of 560 nm, which is generated when luciferin is oxidized by luciferase using ATP.

***ATP*+*Luciferin*+*O*₂ → *AMP*+*PPᵢ*+*CO*₂+*Light*(560*nm*)**

Currently, there are products developed using the aforementioned luciferin-based ATP analysis method, such as Lumitester available from Kikkoman, EnSURE Luminometer available from Hygiena, ATPlite available from PerkinElmer, and the like. However, expensive optical analysis equipment is required to measure and analyze the colorimetric response signal of luciferin. Therefore, there is a need to develop portable ATP measurement equipment that overcomes price issues with existing equipment and is capable of being conveniently used by the general public.

Currently, one of the most widespread medical devices worldwide is a self-monitoring blood glucose meter. Self-monitoring blood glucose meters are small in size, highly portable, inexpensive, and simple to use, and are thus widely provided to and used by the general public. Recently, a method of detecting nucleic acids and the like other than blood glucose using such a self-monitoring blood glucose meter has been developed (US 8,945,943). The above technology extends the utility of self-monitoring blood glucose meters, which are restricted only to blood glucose measurement, to the detection of biomaterials such as nucleic acids and the like, but requires complicated overall analysis processes such as a process of modifying magnetic nanoparticles and enzymes with an aptamer probe, a sample separation process using magnetic nanoparticles, and the like.

Therefore, the inventors of the present invention had made intensive efforts to develop, as a convenient and novel method, a method capable of detecting ATP using a self-monitoring blood glucose meter. As a result, the inventors confirmed that, when an enzyme chain reaction using, as substrates, ATP and glucose, which is a detection indicator, is performed, a reaction in which ATP present in a sample is converted into ADP and then regenerated into ATP continues so that glucose is phosphorylated, and the resultant decrease in glucose concentration can be conveniently measured using a self-monitoring blood glucose meter, thus completing the present invention.

WO2015/069872 A2 is related to Personal glucose meters for detection and quantification of enzymes and metabolites based on coenzyme detection.

### [Summary]

Therefore, the present disclosure has been made in view of the above problems, and it is an object of the present disclosure to provide a method of conveniently detecting ATP by inducing a change in glucose concentration through glucose phosphorylation, an ATP regeneration reaction, and an enzyme chain reaction and measuring the change using a commercially available self-monitoring blood glucose meter.

According to an aspect of the present invention, there is provided a method of detecting or quantifying ATP in a sample, the method comprising (a) adding a sample to be detected to a composition for detecting ATP including glucose, NADP, phosphoenolpyruvic acid, hexokinase, and pyruvate kinase, and then converting glucose in the sample into glucose-6-phosphate and (b) measuring a concentration of the glucose using a blood glucose meter to detect or quantify ATP in the sample.

### [Brief Description of Drawings]

The above and other objects, features and other advantages of the present invention will be more clearly understood from the following detailed description taken in conjunction with the accompanying drawings, in which:
FIG. 1 illustrates a method of analyzing ATP by measuring a change in glucose concentration caused by an enzyme chain reaction using a self-monitoring blood glucose meter, according to the present invention;
FIG. 2 illustrates the results of measuring glucose concentration, which varies depending on the presence or absence and combination of hexokinase and pyruvate kinase using a self-monitoring blood glucose meter;
FIG. 3 illustrates the experimental results of confirming changes in glucose concentration when conducting an experiment using ATP analogues (CTP, GTP, and UTP) as analytes; and
FIG. 4 illustrates the experimental results of confirming changes in glucose concentration when analyzing different concentrations of ATP using an ATP analysis technique based on signal measurement of a self-monitoring blood glucose meter.

### [Detailed Description and Exemplary Embodiments]

Unless defined otherwise, technical and scientific terms used herein have the same meanings as those commonly understood by one of ordinary skill in the art to which the present invention pertains. Generally, the nomenclature used herein is well known in the art and commonly used.

A method of confirming the presence or absence of ATP present in a sample and quantifying ATP by measuring a decrease in glucose concentration through conversion of ATP present in a sample into ADP and regeneration into ATP and the consequent glucose phosphorylation reaction using a portable self-monitoring blood glucose meter has been developed for the present invention.

A technique for quantitatively analyzing ATP has been developed using a glucose concentration reduction phenomenon caused by the production of glucose-6-phosphate through transfer, to glucose, of a phosphate group, which is released by conversion of ATP into ADP by hexokinase for the present invention.

Therefore, the present invention relates to a method of detecting or quantifying ATP present in a sample, including: (a) adding a sample to be detected to a composition for detecting ATP including glucose, NADP, phosphoenolpyruvic acid, hexokinase, and pyruvate kinase, and then converting glucose in the sample into glucose-6-phosphate; and (b) measuring the concentration of the glucose using a blood glucose meter to detect or quantify ATP in the sample.

Process (a) of the present invention includes the following reaction processes:
(i) inducing a reaction in which ATP is converted into ADP by hexokinase and glucose is converted into glucose-6-phosphate; (ii) inducing a reaction in which ADP is converted into ATP by pyruvate kinase and phosphoenolpyruvic acid is converted into pyruvate; (iii) recycling ATP using an enzyme chain reaction induced by a combination of hexokinase and pyruvate kinase; and (iv) inducing a decrease in glucose concentration through the enzyme chain reaction.

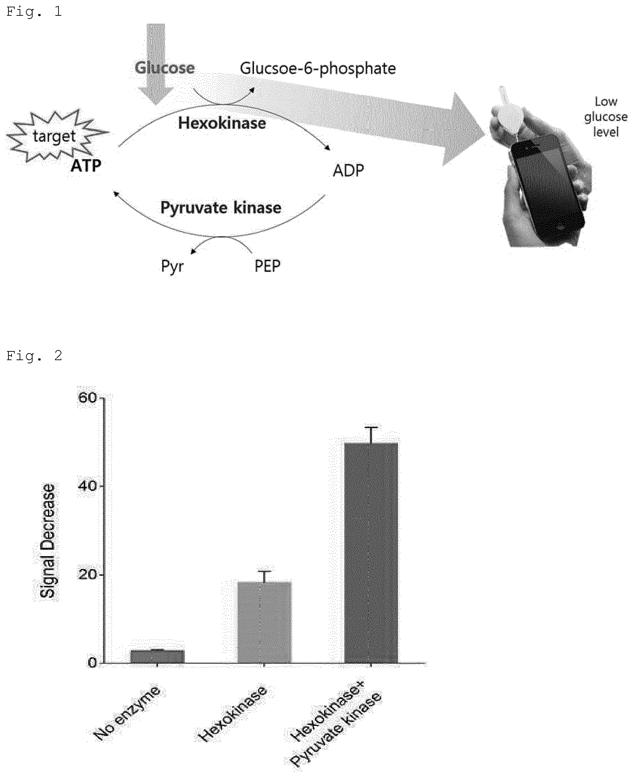

In process (b) of the present invention, the concentration of glucose in the sample, reduced through reaction (a) above, is measured using a self-monitoring blood glucose meter, and ATP in the sample is detected and quantified through the change in glucose concentration measured using the self-monitoring blood glucose meter (see FIG. 1).

In the present invention, glucose-6-phosphate is produced by transferring, to glucose, a phosphate group released by conversion of ATP present in the sample into ADP by hexokinase, resulting in reduced glucose concentration, and ATP may be quantified using this.

In the present invention, the composition for detecting ATP include NADP and phosphoenolpyruvic acid.

In the present invention, the glucose concentration is measured using a blood glucose meter.

According to the present invention, ATP is converted into ADP by hexokinase and glucose is converted into glucose-6-phosphate. At the same time, ADP produced by the hexokinase enzyme reaction is converted back into ATP by pyruvate kinase, and phosphoenolpyruvic acid is converted into pyruvate. The enzyme chain reaction is induced by a combination of the hexokinase enzyme reaction and the pyruvate kinase enzyme reaction. In the case in which neither of the two enzymes is present in the sample, the enzyme chain reaction is unable to proceed, and thus no change in glucose concentration occurs. In contrast, in the case in which both enzymes are present, ATP is recycled and the glucose concentration decreases. Moreover, when only the hexokinase enzyme is present in the enzyme chain reaction, ATP is converted into ADP, resulting in reduced glucose concentration, but only a small amount of glucose is reduced since ATP is unable to be recycled. From these phenomena, it was verified that ATP was recycled through the enzyme chain reaction using a combination of hexokinase and pyruvate kinase according to the present invention and that the presence or absence of ATP could be determined by measuring the glucose concentration using a self-monitoring blood glucose meter (see FIG. 2).

Hereinafter, the present invention will be described in further detail with reference to the following examples. It will be obvious to those of ordinary skill in the art that these examples are provided for illustrative purposes only and are not intended to limit the scope of the present invention.

### Example 1. Induction of Enzyme Chain Reaction for Detection and Quantification of ATP

19 µL of a reaction buffer solution of an enzyme chain reaction for the detection and quantification of ATP was prepared. The reaction buffer solution contained 5 µL of D-glucose (50-400 mM), 5 µL of MgCl₂ (5-500 mM), 5 µL of Tris-HCl (0.5-3 M, pH 7.4), 1 µL of 50mM β-NADP, 1 µL of 100 mM phosphoenolpyruvic acid, and 2 µL of DW. For the enzyme chain reaction, 11 µL of an enzyme mixture of 5 units of hexokinase, 5 units of pyruvate kinase, and 0.4 unit of glucose-6-phosphate dehydrogenase was prepared. 20 µL of an ATP-containing analysis sample was added to a mixture of the prepared reaction buffer solution and the enzyme mixture, and the resulting mixture was stored at 30 °C for 30 minutes to induce a glucose conversion reaction through an enzyme chain reaction using ATP as a substrate. After the reaction, the concentration of glucose in the sample was analyzed using a self-monitoring blood glucose meter.

### Example 2. Verification of Selectivity and Sensitivity of ATP Detection Using Enzyme Chain Reaction

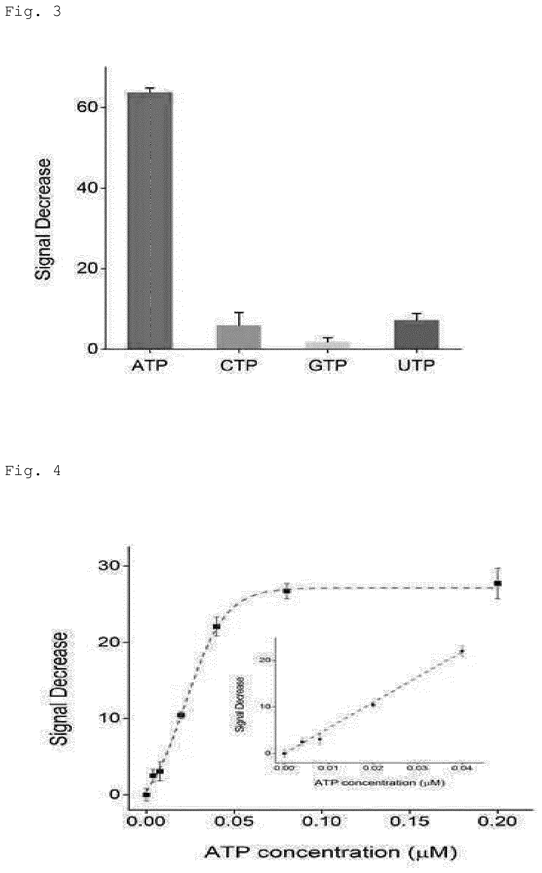

Instead of 20 µL of the ATP-containing analysis sample used in Example 1, 20 µL of a 10 µM analysis sample containing cytidine 5'-triphosphate (CTP), guanosine 5'-triphosphate (GTP), and uridine 5'-triphosphate (UTP), which are ATP analogues, was prepared to perform an enzyme chain reaction as in Example 1, and as a result of measuring glucose concentration using a self-monitoring blood glucose meter, a significant change in glucose concentration was found only in the ATP-containing analysis sample (see FIG. 3). In addition, variation in glucose concentration after the enzyme chain reaction depending on the concentration of ATP included in the analysis sample used in Example 1 was measured using a self-monitoring blood glucose meter, thereby analyzing the concentration of ATP in the sample. 20 µL of analysis samples containing various concentrations (0 nM, 4 nM, 8 nM, 20 nM, 40 nM, 80 nM, and 200 nM) of ATP were prepared to perform an enzyme chain reaction as in Example 1, and as a result of measuring varying glucose concentration using a self-monitoring blood glucose meter, it was confirmed that the limit of detection (LOD) of ATP was 4.9 nM (see FIG. 4).

### [Industrial Applicability]

As is apparent from the above description, a method of detecting ATP using a self-monitoring blood glucose meter, according to the present invention, is inexpensive, can be easily and conveniently used by anyone, and uses a commercially available self-monitoring blood glucose meter, enabling rapid market penetration to thus replace an existing ATP analysis method based on a colorimetric response signal, and thus may be utilized for rapid microorganism detection by catering companies or in institutional food service processes.

While the present invention has been particularly shown and described with reference to specific embodiments thereof, it will be obvious to those of ordinary skill in the art that these detailed descriptions are merely exemplary embodiments and are not intended to limit the scope of the present invention. Therefore, the actual scope of the present invention will be defined by the appended claims.

The present invention was conducted as part of the mid-career researcher support project (2015R1A2A1A01005393) of the National Research Foundation of Korea.

## Claims

1. A method of detecting or quantifying ATP in a sample, the method comprising:
(a) adding a sample to be detected to a composition for detecting ATP comprising glucose, NADP, phosphoenolpyruvic acid, hexokinase, and pyruvate kinase, and then converting glucose in the sample into glucose-6-phosphate; and
(b) measuring a concentration of the glucose using a blood glucose meter to detect or quantify ATP in the sample.

2. The method according to claim 1, wherein the composition for detecting ATP further comprises glucose-6-phosphate dehydrogenase.

## Patentansprüche

1. Verfahren zum Nachweis oder zur Quantifizierung von ATP in einer Probe, wobei das Verfahren Folgendes umfasst:
(a) Zugeben einer Nachweisprobe zu einer Zusammensetzung zum Nachweis von ATP, umfassend Glucose, NADP, Phosphoenolpyruvinsäure, Hexokinase und Pyruvatkinase, und dann Überführen von Glucose in der Probe in Glucose-6-phosphat; und
(b) Messen der Konzentration der Glucose unter Verwendung eines Blutglucosemessgeräts, um ATP in der Probe nachzuweisen oder zu quantifizieren.

2. Verfahren nach Anspruch 1, wobei die Zusammensetzung zum Nachweis von ATP weiters Glucose-6-phosphatdehydrogenase umfasst.

## Revendications

1. Procédé de détection ou de quantification d'ATP dans un échantillon, le procédé comprenant les étapes consistant à :
(a) ajouter un échantillon à détecter à une composition de détection d'ATP contenant du glucose, du NADP, de l'acide phosphoénolpyruvique, de l'hexokinase et de la pyruvate kinase, puis convertir le glucose dans l'échantillon en glucose-6-phosphate ; et
(b) mesurer une concentration de glucose en utilisant un glucomètre pour détecter ou quantifier l'ATP dans l'échantillon.

2. Procédé selon la revendication 1, dans lequel la composition de détection d'ATP comprend de la glucose-6-phosphate déshydrogénase.
